# EUROPEAN PATENT SPECIFICATION

(11) **EP 3 166 681 B1**
(45) Date of publication and mention of the grant of the patent: **19.12.2018**
(21) Application number: 15739147.5
(22) Date of filing: 06.07.2015
(51) Int. Cl.: A61L 29/16, A61K 31/155, A61M 25/00, A61M 39/00, A61M 39/16, A61M 39/02, A61M 39/26

(54) **PORTED CATHETER HAVING AN ANTIMICROBIAL ACTUATOR FOR OPENING THE SIDE PORT OF THE PORTED CATHETER**
PORTIERTER KATHETER MIT EINEM ANTIMIKROBIELLEN AKTUATOR ZUM ÖFFNEN DES SEITLICHEN ANSCHLUSSES DES PORTIERTEN KATHETERS
CATHÉTER À ORIFICES AVEC UN ACTIONNEUR ANTIMICROBIEN POUR OUVRIR L'ORIFICE LATÉRAL DU CATHÉTER À ORIFICES

(30) Priority: 08.07.2014 US 201414326072
(43) Date of publication of application: 17.05.2017
(73) Proprietor: Becton, Dickinson and Company, Franklin Lakes, NJ 07417-1880 (US)
(72) Inventor: HARDING, Weston F., Lehi, Utah 84043 (US)
(74) Representative: dompatent von Kreisler Selting Werner - Partnerschaft von Patent- und Rechtsanwälten mbB
(86) International application number: PCT/US2015/039262
(87) International publication number: WO 2016/007440

(56) References cited:
- EP-A1- 0 070 087
- EP-A2- 0 370 997
- WO-A1-96/40359
- WO-A1-99/36490
- WO-A1-99/43971
- WO-A1-2011/118680
- WO-A2-2011/048204
- US-A- 4 925 668
- US-A1- 2013 245 568

## Description

### BACKGROUND OF THE INVENTION

The present invention relates generally to inserts for medical devices that are configured to elute an antimicrobial agent. In particular, an actuator for a side port of a ported catheter can be configured to elute an antimicrobial agent to disinfect the side port including any fluid contained within the side port.

Catheters are commonly used for a variety of infusion therapies. For example, catheters are used for infusing fluids, such as normal saline solution, various medicaments, and total parenteral nutrition into a patient, withdrawing blood from a patient, as well as monitoring various parameters of the patient's vascular system.

Catheter-related bloodstream infections are caused by the colonization of microorganisms in patients with intravascular catheters and I.V. access devices. These infections are an important cause of illness and excess medical costs. More importantly, these infections often result in patient deaths.

Many techniques have been employed to reduce the risk of infection from a catheter or other intravenous device. For example, catheters have been designed that employ an antimicrobial lubricant or an antimicrobial coating on an inner or outer surface of the catheter. Similarly, antimicrobial lubricants or coatings have been applied to the surfaces of other components of a catheter assembly, components attached to the catheter assembly, or other medical devices which may come in direct contact with the patient's vasculature or in contact with a fluid that may enter the patient's vasculature. Further, some devices or components are made of a material that is impregnated with an antimicrobial agent.

Although these techniques have been beneficial, there are various drawbacks that limit their usefulness. For example, it can be difficult and/or expensive to apply an antimicrobial coating or lubricant to the complex internal and external geometries of many devices or components. Also, some devices or components are preferably made of a material that is not suitable for the application of an antimicrobial coating or that cannot be impregnated with an antimicrobial agent. Because of such difficulties, the current techniques for providing antimicrobial protection are oftentimes not used or, if used, are not adequately applied to provide maximum antimicrobial protection.

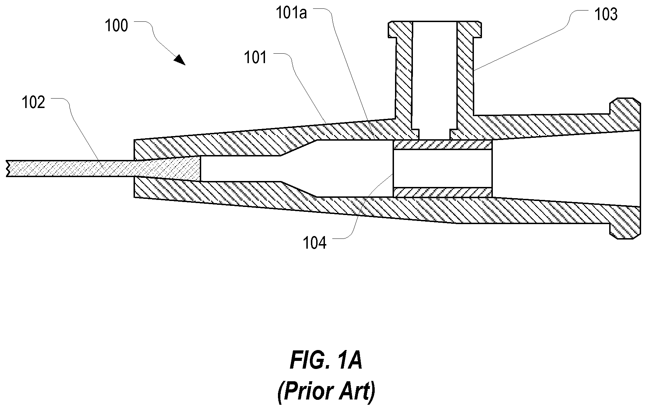

Catheters with side ports (commonly referred to as ported catheters) are oftentimes used because additional bolus medications can be easily injected into the catheter adapter via the side port. An example of a typical ported catheter 100 is shown in Figures 1A-1C. As shown, ported catheter 100 comprises a catheter adapter 101 having a side port 103 and a catheter 102 that extends from the distal end of the catheter adapter. A valve for the side port 103 is commonly formed using a piece of tubing 104 positioned within the inner lumen 101a of the catheter adapter 101. The piece of tubing 104 is made of a resilient material and has an external diameter at least as large as the inner diameter of the inner lumen 101a so that the tubing 104 seals the inner lumen 101a from the side port 103.

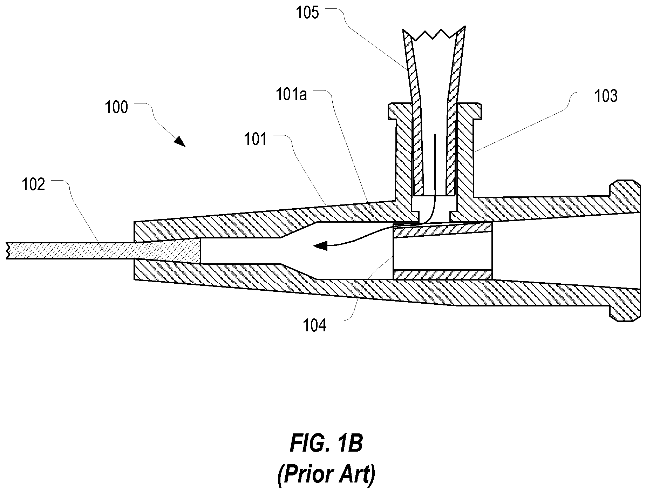

Figure 1B illustrates how tubing 104 is displaced to open a flowpath through the side port 103 into the inner lumen 101a. As shown, a separate device 105 (e.g. a luer connector) can be inserted into side port 103. Fluid can then be expelled from device 105. The pressure built up within side port 103 as the fluid is injected into side port 103 causes tubing 104 to collapse inwardly as shown in Figure 1B. The inward collapse of tubing 104 creates the flowpath through which fluid may flow from device 105 and into lumen 101a as indicated by the arrow.

Various problems exist with this type of ported catheter. For example, as the fluid is ejected from device 105 and prior to tubing 104 collapsing, a substantial amount of pressure can build within side port 103. This pressure is necessary to cause tubing 104 to collapse. However, in some instances, if the pressure becomes too high, it can cause device 105 to separate from side port 103 allowing fluid to spray out from side port 103.

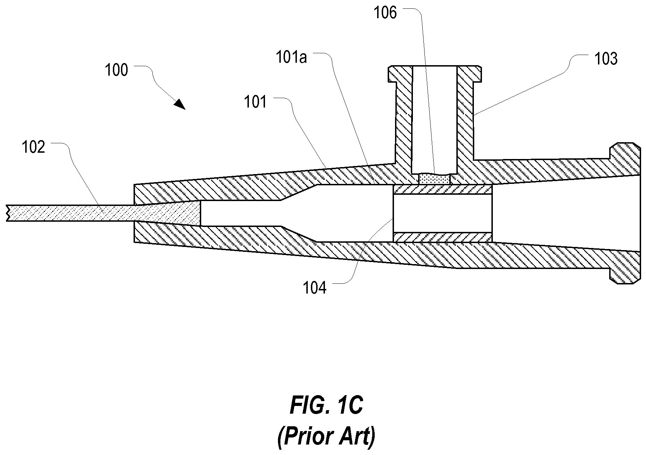

Another problem that exists with common ported catheters is that, after fluids are injected via side port 103, some residual fluid will remain within side port 103 on top of tubing 104. Figure 1C represents the state of the ported catheter 100 after device 105 has been removed from side port 103. As shown, once fluid is no longer injected from device 105, the lack of pressure will allow tubing 104 to snap back to its original position thereby sealing the opening into inner lumen 101a. When this occurs, fluid 106 remains within side port 103. This residual fluid 106 cannot effectively be removed from side port 103. If side port 103 is not sealed after use, fluid 106 can quickly become contaminated. Then, when side port 103 is again used for infusion, the contaminated fluid 106 will be flushed into lumen 101a and ultimately into the patient thereby increasing the risk of infection.

A further problem that exists with common ported catheters is that they only allow for fluid flow in a single direction. Because external pressure from fluid flowing into lumen 101a is required to cause tubing 104 to collapse inwardly to open the flowpath, it is not possible to have fluid within inner lumen 101a (e.g. a patient's blood) flow out through side port 103.

WO 99/43971 discloses a ported catheter (flush valve for catheter) comprising:
a catheter adapter having an inner lumen;
a catheter extending distally from the catheter adapter;
a side port forming an opening through a sidewall of the catheter adapter into the inner lumen;
a tubing positioned within the inner lumen to cover the opening formed by the side port; and
an actuator contained within the side port, the actuator being configured to compress the tubing inwardly when a device is inserted into the side port, the inward compression of the tubing opening a flowpath from the side port into the inner lumen.

WO 96/40359 discloses a ported catheter comprising:
a catheter adapter having an inner lumen;
a catheter extending distally from the catheter adapter;
a side port forming an opening through a sidewall of the catheter adapter into the inner lumen;
a tubing positioned within the inner lumen to cover the opening formed by the side port; and
an actuator contained within the side port, the actuator being configured to compress the tubing inwardly when a device is inserted into the side port, the inward compression of the tubing opening a flowpath from the side port into the inner lumen.

### BRIEF SUMMARY OF THE INVENTION

The present invention extends to ported catheters that contain actuators within their side ports. These actuators comprises one or more antimicrobial agent(s) and can be comprised of a material or contain a coating that elutes the antimicrobial agent(s) when the actuator comes in contact with a fluid. Therefore, any residual fluid that remains within the side port after infusion can be disinfected by the antimicrobial agent eluted from the actuator.

The use of an actuator in the side port also facilitates bidirectional fluid flow through the side port. The actuator can be configured to open a flowpath when an external device is inserted into the side port. Accordingly, the flowpath can be opened without requiring the presence of built-up pressure within the side port.

According to the present invention, the actuator is implemented in a ported catheter.

In particular, the present invention relates to:
1. A ported catheter comprising:
   a catheter adapter having an inner lumen;
   a catheter extending distally from the catheter adapter;
   a side port forming an opening through a sidewall of the catheter adapter into the inner lumen;
   a tubing positioned within the inner lumen to cover the opening formed by the side port; and
   an actuator contained within the side port, the actuator being configured to compress the tubing inwardly when a device is inserted into the side port, the inward compression of the tubing opening a flowpath from the side port into the inner lumen, characterized in that the actuator comprises one or more antimicrobial agent(s), wherein the actuator comprises a lumen through which fluid may flow and a bottom portion having a diameter that is smaller than the diameter of the opening within the side port and a top portion having a diameter that is larger than the diameter of the opening within the side port and wherein the antimicrobial agent(s) elute into a fluid when the fluid contacts the actuator.
2. The ported catheter of embodiment 1, wherein the antimicrobial agent is contained within a material from which the actuator is formed, preferably wherein the material is a UV curable material, more preferably wherein the material comprises a coating on a surface of another material from which the actuator is formed.
3. The ported catheter of embodiment 2, wherein the antimicrobial agent comprises chlorhexidine diacetate.
4. The ported catheter of embodiment 2, wherein the antimicrobial agent is contained within a lubricant applied to a surface of the actuator, preferably wherein the antimicrobial agent comprises one or more of chlorhexidine diacetate or chlorhexidine gluconate, more preferably wherein the lubricant comprises fused silica.
5. The ported catheter of embodiment 1, wherein the actuator comprises a lumen through which fluid flows from the device into the inner lumen.
6. The ported catheter of embodiment 1, wherein the actuator comprises a bottom portion that extends into the inner lumen when the device is inserted into the side port.
7. The ported catheter of embodiment 1, wherein, when the device is removed from the side port, the tubing forces the actuator back into the side port to allow the tubing to seal the opening formed by the side port.
8. The ported catheter of embodiment 1, wherein the side port and the actuator are configured to prevent the actuator from being removed from the side port.

Additional features and advantages of the invention will be set forth in the description which follows, and in part will be obvious from the description, or may be learned by the practice of the invention. The features and advantages of the invention may be realized and obtained by means of the instruments and combinations particularly pointed out in the appended claims. These and other features of the present invention will become more fully apparent from the following description and appended claims, or may be learned by the practice of the invention as set forth hereinafter.

### BRIEF DESCRIPTION OF THE DRAWINGS

In order to describe the manner in which the above-recited and other advantages and features of the invention can be obtained, a more particular description of the invention briefly described above will be rendered by reference to specific embodiments thereof which are illustrated in the appended drawings. Understanding that these drawings depict only typical embodiments of the invention and are not therefore to be considered to be limiting of its scope, the invention will be described and explained with additional specificity and detail through the use of the accompanying drawings in which:

Figures 1A-1C illustrate a cross-sectional view of a prior art ported catheter. The prior art ported catheter includes tubing within the lumen of the catheter that is compressed inwardly when sufficient pressure is built up within the side port.

Figures 2A-2C illustrate a cross-sectional view of a ported catheter in accordance with one or more embodiments of the present invention. The ported catheter in accordance with embodiments of the present invention includes an actuator that compresses the tubing when a device is attached to the side port.

Figures 3A-3C illustrate detailed views of the actuator shown in Figures 2A-2C respectively.

### DETAILED DESCRIPTION OF THE INVENTION

The present invention extends to ported catheters that contain actuators within their side ports. These actuators comprises one or more antimicrobial agent(s) and can be comprised of a material or contain a coating that elutes the antimicrobial agent(s) when the actuator comes in contact with a fluid. Therefore, any residual fluid that remains within the side port after infusion can be disinfected by the antimicrobial agent eluted from the actuator.

The use of an actuator in the side port also facilitates bidirectional fluid flow through the side port. The actuator can be configured to open a flowpath when an external device is inserted into the side port. Accordingly, the flowpath can be opened without requiring the presence of built-up pressure within the side port.

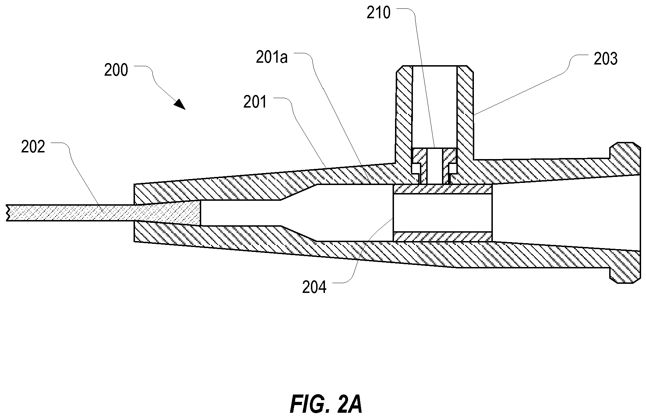

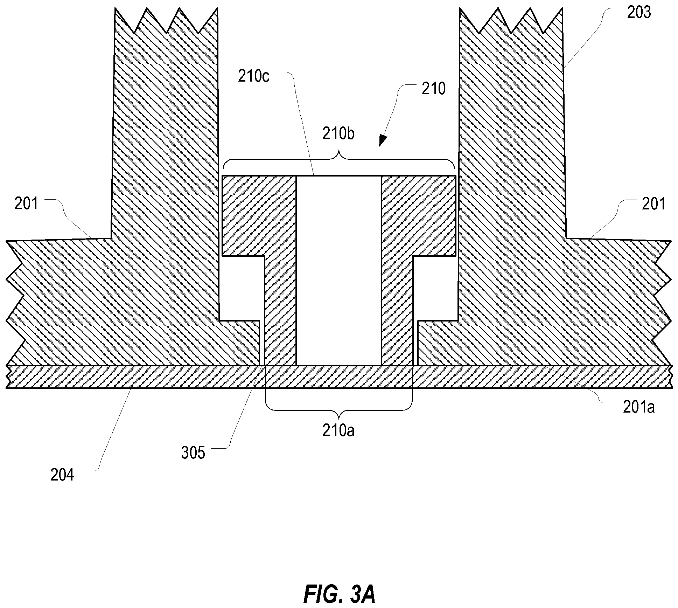

Figures 2A-2C illustrate an example of a ported catheter 200 that employs an actuator 210 to open and disinfect the side port 203 of the ported catheter. Figures 3A-3C illustrate detailed views of the actuator 210 within side port 203 and correspond to Figures 2A-2C respectively. As shown, ported catheter 200 comprises a catheter adapter 201 having an inner lumen 201a. A side port 203 extends from the catheter adapter 201 and forms an opening into the inner lumen 201a. This opening is sealed by a piece of tubing 204 positioned within the inner lumen 201a as was described with reference to Figures 1A-1C.

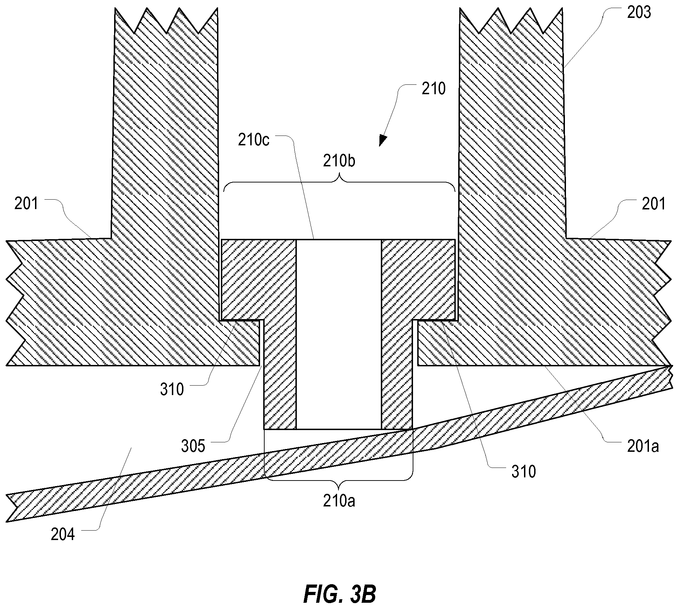

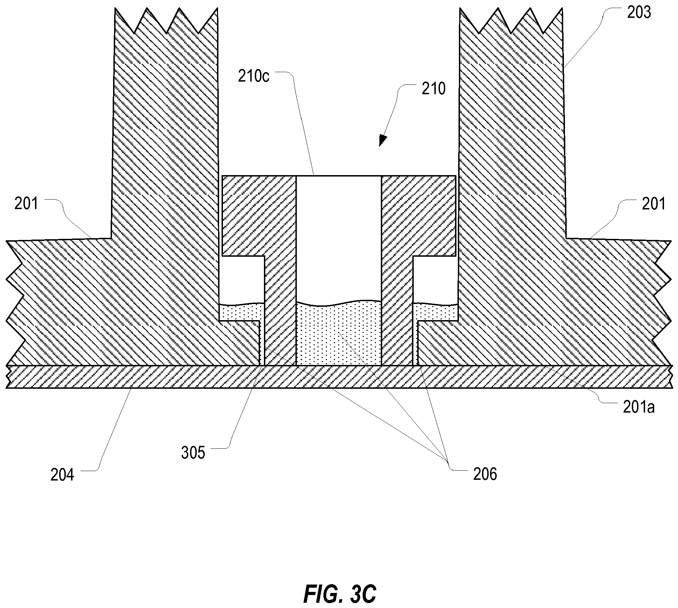

Unlike ported catheter 100, ported catheter 200 includes an actuator 210 positioned within side port 203. As better shown in Figure 3A, actuator 210 comprises a bottom portion 210a having a diameter that is smaller than the diameter of the opening within side port 203 (shown as 305 in Figure 3) and a top portion 210b having a diameter that is larger than the diameter of the opening within side port 203. Actuator 210 also includes a lumen 210c through which fluid may flow. As best shown in Figures 3A and 3B, side port 203 can include ridges 310 (forming opening 305) which prevent actuator 210 from passing completely through opening 305.

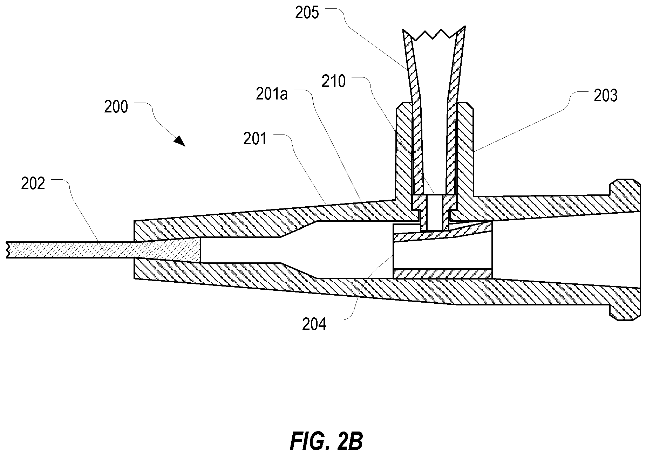

Referring now to Figures 2B and 3B, when a device 205 is inserted into side port 203, the tip of device 205 can force actuator 210 against tubing 204 causing tubing 204 to collapse inwardly. As best seen in Figure 3B, the collapsing of tubing 204 creates a flowpath through actuator 210 and into lumen 201a. In some embodiments, the bottom portion 210a can include one or more channels or openings (in addition to the opening formed by lumen 210c) through which fluid may pass out from actuator 210 and into lumen 201a. For example, the bottom portion 210a can include one or more channels that extend upwardly from the bottom tip or one or more holes through the bottom portion 210a.

It is noted that the collapsing of tubing 204 can be accomplished entirely from the force applied by actuator 210 to tubing 204 and therefore no fluid pressure needs to be built up to cause tubing 204 to collapse. For this reason, the use of actuator 210 minimizes the likelihood that any fluid will be sprayed out from side port 203.

Additionally, because the flowpath around tubing 204 is formed by actuator 210 and not by pressure built-up within side port 203, the use of actuator 210 allows fluid to flow bidirectionally within side port 203. In other words, because actuator 210 will maintain the flowpath from side port 203 into lumen 201a even when no fluid is flowing out from device 205, device 205 can be used to collect fluid from within lumen 201a. For example, if device 205 is a syringe, the syringe can be used to collect blood from within lumen 201a.

In some implementations, side port 203 and/or device 205 can be modified (not shown) to allow device 205 to be interlocked within side port 203. This may be desired in situations where fluid will be injected from device 205 at high pressure to prevent the forces generated by the high pressure injection (i.e. forces caused when the fluid exists device 205) from causing device 205 to back out from side port 203. However, in many implementations, no locking between device 205 and side port 203 is required because the flowpath created when actuator 210 compresses tubing 204 enables fluid flow without the buildup of pressure.

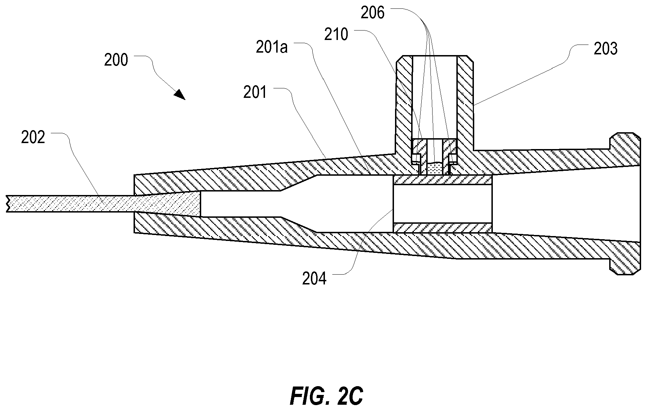

Referring now to Figures 2C and 3C, once the injection of fluid has been completed and device 205 has been removed from side port 203, the resiliency of tubing 204 will cause tubing 204 to return to its original position thereby forcing actuator 210 back out of lumen 201a. At this point, tubing 204 again forms a seal between lumen 201a and side port 203. Once this seal is formed, residual fluid 206 will remain within side port 203. Actuator 210 can be configured so that it remains positioned within side port 203 and particularly within opening 305. In this position, actuator 210 will be in contact with residual fluid 206 as shown in Figures 2C and 3C. Various techniques can be employed to maintain actuator 210 within side port 203 such as by forming ridges, channels, or other structure within side port 203 and/or actuator 210 that limit the upward movement of actuator 210.

In some embodiments of the invention, actuator 210 can be comprised of a material or contain a coating that elutes antimicrobial agents when actuator 210 is in contact with a fluid. In such cases, as fluid 206 contacts actuator 210, the antimicrobial agent contained within or on actuator 210 will elute into fluid 206 thereby maintaining the sterility of fluid 206 as well as the sterility of surfaces within side port 203. By maintaining the sterility of side port 203, the likelihood that microbes will be introduced through side port 203 during a subsequent infusion is reduced.

Antimicrobial actuators in accordance with one or more embodiments of the invention can be comprised of a base material matrix and one or more antimicrobial agents. In some embodiments, the base material matrix can be a UV curable, hydrophilic material that contains an antimicrobial agent with controlled release (elution) characteristics. Alternatively, a base material can be coated with an antimicrobial coating from which an antimicrobial agent will elute when subject to a fluid. Examples of materials that could be used to form the antimicrobial actuator of the present invention include those disclosed in U.S. Patent No.: 8,512,294 titled Vascular Access Device Antimicrobial Materials And Solutions; U.S. Patent Application No.: 12/397,760 (published as US 2010-0135949) titled Antimicrobial Compositions; U.S. Patent Application No.: 12/476,997 (published as US 2010-0137472) titled Antimicrobial Coating Compositions; U.S. Patent Application No.: 12/490,235 (published as US 2010-0136209) titled Systems And Methods For Applying An Antimicrobial Coating To A Medical Device; and U.S. Patent Application No.: 12/831,880 (published as US 2011-0009831) titled Antimicrobial Coating For Dermally Invasive Devices.

In one particular embodiment, the antimicrobial agent used to form an actuator can be chlorhexidine including chlorhexidine diacetate (CHA) and chlorhexidine gluconate (CHG). However, any other antimicrobial agent that will elute from a base material or from a coating on a base material could be used. Any material having elution characteristics can be employed as the base material of an actuator. Examples of suitable materials include UV cured acrylate-urethanes and heat-cured polymers which soften in water, such as hygroscopic polyurethanes. Also, if an antimicrobial lubricant is employed to provide antimicrobial agents, the actuator can be formed of any suitable material on which the lubricant can be applied whether or not it provides elution characteristics.

The amount of antimicrobial agent employed within a base material matrix or a lubricant coating can be varied to provide a desired mechanical property or elution characteristic. For example, in some instances a matrix is provided which comprises solid antimicrobial agent particles in an amount representing approximately 0.1-40% w/w of the matrix. These particles may range in size from 100 nm (fine powder) to 0.15 mm (salt-sized crystals). Additional additives may also be used to attain a particular characteristic. These additional additives include: multiple antimicrobial agents to widen the spectrum of microbes that will be affected; viscosity modifiers such as silica; color modifiers such as dyes or titanium dioxide; strength or stiffness modifiers such as glass fibers, ceramic particles such as zirconia, or metallic fibers; radiopacity modifiers such as barium sulfate; and magnetic susceptibility enhancers such as gadolinium chelates.

In some embodiments, a matrix can be used to form a coating on another material of the actuator. In such cases, the matrix can comprise 9% chlorhexidine diacetate (or chlorhexidine gluconate) mixed in a UV-curable acrylate adhesive (e.g. mCAST 7104 manufactured by Electronic Materials, Inc. or Breckenridge, CO).

In embodiments where a lubricant coating containing the antimicrobial agent is used, the lubricant coating can comprise 9% chlorhexidine diacetate or chlorhexidine gluconate mixed with MED-460 silicone lube. The viscosity of the lube can be modified by adding fumed silica in concentrations up to 3%. The use of 9% chlorhexidine represents specific examples; however, other percentages could equally be used to provide a desired elution duration.

To summarize, an antimicrobial actuator in accordance with the invention can be molded out of any material and then coated with an antimicrobial eluting coating or lubricant, or can be cast or formed out of a base material matrix that incorporates the antimicrobial agent. Regardless of how the actuator is formed or the materials used to form it, an actuator in accordance with the present invention can elute antimicrobial agents into fluid to sterilize or maintain the sterility of the fluid and contacting surfaces.

Because actuator 210 can include antimicrobial agents to sterilize fluid contained within side port 203, the present invention minimizes the likelihood of infection when ported catheter 200 is used. In some embodiments, when side port 203 is not in use, a cap or other cover can be placed over side port 203 to prevent contaminants from entering side port 203. However, because actuator 210 can provide antimicrobial benefits, a cap or other cover may not be required or may not need to provide any level of antimicrobial protection to side port 203. Accordingly, actuator 210 can facilitate the aseptic use of a ported catheter.

## Claims

1. A ported catheter (200) comprising:
a catheter adapter (201) having an inner lumen (201a);
a catheter extending distally from the catheter adapter (201);
a side port (203) forming an opening through a sidewall of the catheter adapter (201) into the inner lumen (201a);
a tubing (204) positioned within the inner lumen (201a) to cover the opening formed by the side port (203); and
an actuator (210) contained within the side port (203), the actuator (210) being configured to compress the tubing (204) inwardly when a device (205) is inserted into the side port (203), the inward compression of the tubing (204) opening a flowpath from the side port (203) into the inner lumen (201a), **characterized in that** the actuator (210) comprises one or more antimicrobial agent(s), wherein the actuator (210) comprises a lumen (210c) through which fluid may flow and a bottom portion (210a) having a diameter that is smaller than the diameter of the opening within the side port (203) and a top portion (210b) having a diameter that is larger than the diameter of the opening within the side port (203) and wherein the antimicrobial agent(s) elute into a fluid when the fluid contacts the actuator (210).

2. The ported catheter (200) of claim 1, wherein the antimicrobial agent is contained within a material from which the actuator (210) is formed, preferably wherein the material is a UV curable material, more preferably wherein the material comprises a coating on a surface of another material from which the actuator (210) is formed.

3. The ported catheter (200) of claim 2, wherein the antimicrobial agent comprises chlorhexidine diacetate.

4. The ported catheter (200) of claim 2, wherein the antimicrobial agent is contained within a lubricant applied to a surface of the actuator (210), preferably wherein the antimicrobial agent comprises one or more of chlorhexidine diacetate or chlorhexidine gluconate, more preferably wherein the lubricant comprises fused silica.

5. The ported catheter (200) of claim 1, wherein the actuator (210) comprises a lumen through which fluid flows from the device (205) into the inner lumen (201a).

6. The ported catheter (200) of claim 1, wherein the actuator (210) comprises a bottom portion (210a) that extends into the inner lumen (201a) when the device (205) is inserted into the side port (203).

7. The ported catheter (200) of claim 1, wherein, when the device (205) is removed from the side port (203), the tubing forces the actuator (210) back into the side port (203) to allow the tubing (204) to seal the opening formed by the side port (203).

8. The ported catheter (200) of claim 1, wherein the side port (203) and the actuator (210) are configured to prevent the actuator (210) from being removed from the side port (203).

## Patentansprüche

1. Katheter mit Seitenanschluss (200), umfassend:
einen Katheteradapter (201) mit einem inneren Lumen (201a);
einen Katheter, der sich in Bezug auf den Katheteradapter (201) distal erstreckt;
einen Seitenanschluss (203), der eine Öffnung durch eine Seitenwand des Katheteradapters (201) in das innere Lumen (201a) bildet;
einen Schlauch (204), der sich innerhalb des inneren Lumens (201a) befindet, um die durch den Seitenanschluss (203) gebildete Öffnung abzudecken; und
ein Stellglied (210), das in dem Seitenanschluss (203) enthalten ist, wobei das Stellglied (210) so konfiguriert ist, dass es den Schlauch (204) nach innen zusammendrückt, wenn eine Vorrichtung (205) in den Seitenanschluss (203) eingesteckt wird, wobei das Nach-innen-Zusammendrücken des Schlauchs (204) einen Strömungsweg ausgehend von dem Seitenanschluss (203) in das innere Lumen (201a) öffnet, **dadurch gekennzeichnet, dass** das Stellglied (210) ein oder mehrere antimikrobielle Mittel, wobei das Stellglied (210) ein Lumen (210c) umfasst, durch das ein Fluid fließen kann, und einen Bodenteil (210a) mit einem Durchmesser, der kleiner ist als der Durchmesser der Öffnung innerhalb des Seitenanschlusses (203), und einen Oberteil (210b) mit einem Durchmesser, der größer ist als der Durchmesser der Öffnung innerhalb des Seitenanschlusses (203), umfasst, wobei das oder die antimikrobiellen Mittel in eine Flüssigkeit eluieren, wenn die Flüssigkeit mit dem Stellglied (210) in Kontakt kommt.

2. Katheter mit Seitenanschluss (200) gemäß Anspruch 1, wobei das antimikrobielle Mittel in einem Material enthalten ist, aus dem das Stellglied (210) gebildet ist, wobei das Material vorzugsweise ein UVhärtbares Material ist, wobei das Material besonders bevorzugt eine Beschichtung auf einer Oberfläche eines anderen Materials, aus dem das Stellglied (210) gebildet ist, umfasst.

3. Katheter mit Seitenanschluss (200) gemäß Anspruch 2, wobei das antimikrobielle Mittel Chlorhexidindiacetat umfasst.

4. Katheter mit Seitenanschluss (200) gemäß Anspruch 2, wobei das antimikrobielle Mittel in einem auf eine Fläche des Stellglieds (210) aufgetragenen Gleitmittel enthalten ist, wobei vorzugsweise das antimikrobielle Mittel Chlorhexidindiacetat und/oder Chlorhexidingluconat umfasst, wobei besonders bevorzugt das Gleitmittel Quarzglas umfasst.

5. Katheter mit Seitenanschluss (200) gemäß Anspruch 1, wobei das Stellglied (210) ein Lumen umfasst, durch das ein Fluid aus der Vorrichtung (205) in das innere Lumen (201a) fließen kann.

6. Katheter mit Seitenanschluss (200) gemäß Anspruch 1, wobei das Stellglied (210) einen Bodenteil (210a) umfasst, der sich in das innere Lumen (201a) hinein erstreckt, wenn die Vorrichtung (205) in den Seitenanschluss (203) eingesteckt wird.

7. Katheter mit Seitenanschluss (200) gemäß Anspruch 1, wobei der Schlauch, wenn die Vorrichtung (205) aus dem Seitenanschluss (203) entfernt wird, das Stellglied (210) zurück in den Seitenanschluss (203) drückt, so dass der Schlauch (204) die durch den Seitenanschluss (203) gebildete Öffnung abdichten kann.

8. Katheter mit Seitenanschluss (200) gemäß Anspruch 1, wobei der Seitenanschluss (203) und das Stellglied (210) so konfiguriert sind, dass sie eine Entfernung des Stellglieds (210) aus dem Seitenanschluss (203) verhindern.

## Revendications

1. Cathéter à orifice (200) comprenant :
un adaptateur de cathéter (201) ayant une lumière interne (201a) ;
un cathéter s'étendant de manière distale à partir de l'adaptateur de cathéter (201) ;
un orifice latéral (203) formant une ouverture à travers une paroi latérale de l'adaptateur de cathéter (201) dans la lumière interne (201a) ;
un tube (204) positionné dans la lumière interne (201a) pour couvrir l'ouverture formée par l'orifice latéral (203) ; et
un actionneur (210) contenu dans l'orifice latéral (203), l'actionneur (210) étant configuré pour comprimer le tube (204) vers l'intérieur lorsqu'un dispositif (205) est inséré dans l'orifice latéral (203), la compression vers l'intérieur du tube (204) ouvrant un trajet d'écoulement depuis l'orifice latéral (203) dans la lumière interne (201a), **caractérisé en ce que** l'actionneur (210) comprend un ou plusieurs agent(s) antimicrobien(s), où l'actionneur (210) comprend une lumière (210c) à travers laquelle un fluide peut s'écouler et une partie inférieure (210a) ayant un diamètre qui est inférieur au diamètre de l'ouverture dans l'orifice latéral (203) et une partie supérieure (210b) ayant un diamètre qui est supérieur au diamètre de l'ouverture dans l'orifice latéral (203) et où l'agent/les agent(s) antimicrobien(s) est/sont élué(s) dans un fluide lorsque le fluide entre en contact avec l'actionneur (210).

2. Cathéter à orifice (200) de la revendication 1, dans lequel l'agent antimicrobien est contenu dans un matériau à partir duquel l'actionneur (210) est formé, de préférence dans lequel le matériau est un matériau durcissable aux UV, plus préférablement dans lequel le matériau comprend un revêtement sur une surface d'un autre matériau à partir duquel l'actionneur (210) est formé.

3. Cathéter à orifice (200) de la revendication 2, dans lequel l'agent antimicrobien comprend du diacétate de chlorhexidine.

4. Cathéter à orifice (200) de la revendication 2, dans lequel l'agent antimicrobien est contenu dans un lubrifiant appliqué sur une surface de l'actionneur (210), de préférence dans lequel l'agent antimicrobien comprend un ou plusieurs parmi le diacétate de chlorhexidine et le gluconate de chlorhexidine, plus préférablement dans lequel le lubrifiant comprend de la silice fondue.

5. Cathéter à orifice (200) de la revendication 1, dans lequel l'actionneur (210) comprend une lumière à travers laquelle un fluide s'écoule du dispositif (205) dans la lumière interne (201a).

6. Cathéter à orifice (200) de la revendication 1, dans lequel l'actionneur (210) comprend une partie inférieure (210a) qui s'étend dans la lumière interne (201a) lorsque le dispositif (205) est inséré dans l'orifice latéral (203).

7. Cathéter à orifice (200) de la revendication 1, dans lequel, lorsque le dispositif (205) est retiré de l'orifice latéral (203), le tube force l'actionneur (210) à revenir dans l'orifice latéral (203) pour permettre au tube (204) de sceller l'ouverture formée par l'orifice latéral (203).

8. Cathéter à orifice (200) de la revendication 1, dans lequel l'orifice latéral (203) et l'actionneur (210) sont configurés pour empêcher l'actionneur (210) d'être retiré de l'orifice latéral (203).
